# EUROPEAN PATENT SPECIFICATION

(11) **EP 3 888 705 B1**
(45) Date of publication and mention of the grant of the patent: **29.04.2026**
(21) Application number: 21164829.0
(22) Date of filing: 25.03.2021
(51) Int. Cl.: A61L 9/12, B65D 83/00, B65D 47/00

(54) **REFILLABLE AIR FRESHENER**
NACHFÜLLBARER LUFTERFRISCHER
DÉSODORISANT RECHARGEABLE

(30) Priority: 31.03.2020 IT 202000006697
(43) Date of publication of application: 06.10.2021
(73) Proprietor: RE.LE.VI. S.p.A., 46040 Rodigo (MN) (IT)
(72) Inventor: Zaniboni, Claudia, 25013 Carpenedolo (IT)
(74) Representative: Perani & Partners S.p.A.

(56) References cited:
- EP-A1- 1 782 842
- JP-A- 2011 201 582
- JP-B2- H07 114 794

## Description

### FIELD OF APPLICATION

The present disclosure relates to an air freshener wherein the liquid freshener composition is refillable.

The present invention relates to a kit employing the aforementioned freshener and a capsule for carrying out a refill of the liquid freshener composition.

The present invention also relates to a method employing such a kit for refilling the air freshener.

### Description of the prior art

Various types of refillable air fresheners are known in the state of the art. Among these, some fresheners comprise a container housing a liquid freshener composition and a cap placed for closing the container. Inside the container there is an absorbing element, such as a wick or a cloth, which is connected in a single piece with the cap and immersed in the freshener composition. By unscrewing the cap and lifting it at least partially, the absorbing element can be removed from the container. In this way the substance impregnated in it evaporates, spreading the fragrance in the surrounding environment.

In a different freshener described in document JP2012217799A, the container has a threaded cap, an absorbing element slidingly connected to the cap in a vertical direction, and a through hole in an upper portion of the cap. The upper portion of the cap is in turn closed by a cover. By opening the cover, a user can lift the absorbing element vertically which, passing through the hole, is exposed so as to diffuse the fragrance in the surrounding environment.

**In** both known fresheners, when the liquid freshener composition is depleted, the user can reuse the container by disengaging the cap from the container and pouring new liquid freshener composition into the container. The latter can be purchased in refill bottles or "stand-up pouches" commonly found on the market, sometimes even sold along with the freshener.

**In** this way it is possible to increase the lifetime of part of the product, i.e., of the container, and avoid waste.

Also document JP2011201582 A shows a refillable freshener which is distinct from the two described above, but which can be coupled to a refill. Such a freshener comprises a container in which a wick is immersed which is connected to the mouth of the container. **In** particular, this freshener does not require the wick to be removed in order to diffuse the fragrance but has a first cap which must be removed for use, and a cover, placed at the mouth of the container and connected to the wick. Specifically, the wick comes out of the cap and faces the outside environment when the first cap is removed, thus allowing the fragrance to diffuse. Such a cover is shaped to be coupled to a mouth of a refill, providing slots that open when the mouth of the refill is coupled to the cover.

The general issue of container reuse is still shared in many areas.

**In** this regard, in addition to the known refill bottles and pouches, capsules for carrying out a refill containing concentrate product that is a liquid, intended to be diluted with water, or a powder, intended to be dissolved in water, have also been recently adopted. These capsules have standard dimensions and are configured to be screwed onto the neck of bottles/flasks of various types, releasing the contents inside them once coupled thereto.

Since the product is concentrated, these capsules have the advantage, compared to bottles and pouches, of allowing a considerable reduction in space, not only in storage but also in transport. As a result, CO₂ emissions decrease significantly for the same number of items transported.

Document JPH07114794 B2 discloses an air freshener comprising a container for a fragrance having a first mouth portion with a cap coupled to said mouth. The cap comprises a lower lid and an upper lid hinged to the lower lid. The lower lid comprises a holding portion to hold an absorbing element which is impregnated by the liquid freshener composition, as well as a second mouth extending from the upper portion of the lower lid and being in fluid communication with the interior of the container.

### Problem of the prior art

Disadvantageously, since in the types of freshener described above the absorbing element is connected to the cap, disengaging the cap from the neck of the container, in order to be able to screw in the refill capsule, implies that the absorbing element is also removed. Accordingly, it is necessary to find a suitable support point for the cap to prevent the absorbing element from coming into contact with dirty surfaces, or from soiling or staining with the freshener composition with which it is soaked. Alternatively, the user, holding it in his hand so as not to have to lay it down, has only one free hand with which to carry out the refill operation.

### SUMMARY OF THE INVENTION

In this context, the technical task underlying the present invention is to provide an air freshener which obviates the drawbacks in the prior art as described above.

In particular, it is an object of the present invention to make available a freshener that is refillable in a quick and easy manner.

A further object of the present invention is to make available a freshener that allows one or more refills to be carried out, while containing transportation costs and space required for storage of the refills.

A further object of the present invention is to make available a kit of an air freshener and a refill capsule configured to carry out a refill of a liquid freshener composition.

A further object of the present invention is to make available a method for refilling a freshener using the aforementioned kit.

The specified technical task and purposes are substantially achieved by a kit, and a method for carrying out a refill comprising the technical features set forth in one or more of the attached claims.

### Advantages of the invention

Thanks to the present invention, it is possible to make a freshener in which a refill can be carried out simply and quickly, and in which this step is more immediate than those known in the state of the art.

Thanks to the present invention, it is also possible to make a refillable freshener in which transportation costs of the fresheners and refills are low, resulting from both reduced fuel consumption and simplified logistics.

### BRIEF DESCRIPTION OF THE DRAWINGS

Further features and advantages of the present invention will appear more clearly from the indicative, and therefore non-limiting, description of a preferred but not exclusive embodiment of a refillable freshener, a kit comprising such a freshener and a capsule configured to carry out a refill, and a method for refilling such a freshener, as illustrated in the attached drawings wherein:

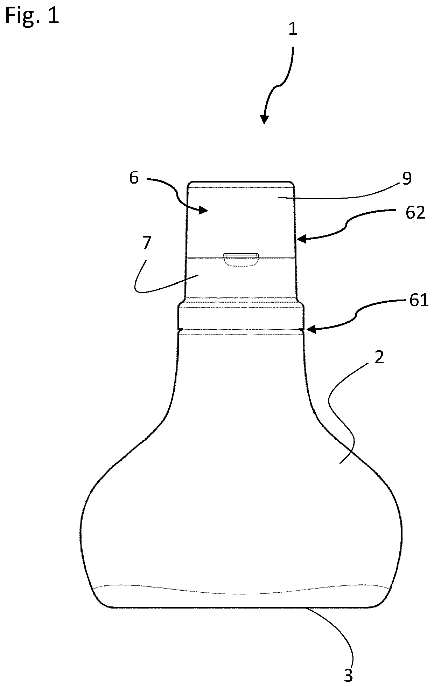

- figure 1 shows an embodiment of the freshener according to the present invention in front view;

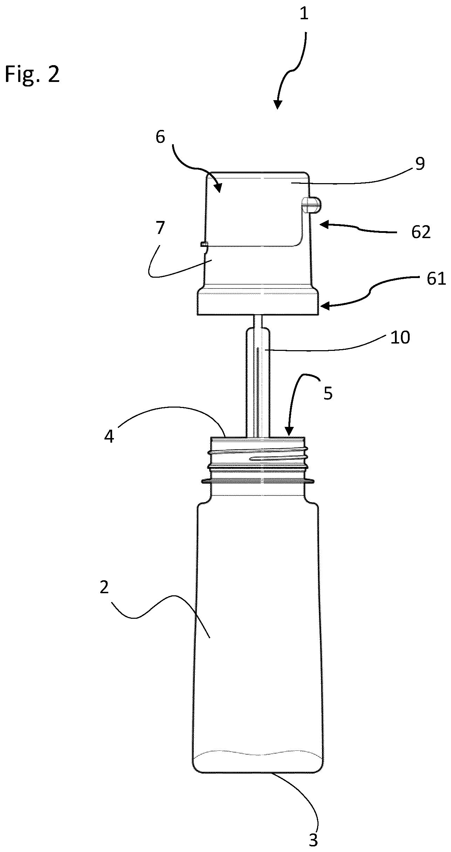

- figure 2 shows the freshener of Figure 1 in use in a side view;
- figure 3 shows a configuration of the freshener of Figure 1 suitable for carrying out a refill step in front view;
- figure 4 shows a detail of the freshener of Figure 1 in a perspective view;
- figure 5 shows the freshener of Figure 1 in a refill step.

### DETAILED DESCRIPTION

With reference to the attached figures, 1 denotes a freshener, and in particular an air freshener.

The air freshener 1 comprises a container 2. Said container 2 comprises a bottom 3 and one first mouth 4. In particular, the container 2 extends in an axial direction between the bottom 3 and the first mouth 4 defining a cavity 5 adapted to house a liquid freshener composition. Preferably, the container 2 is a bottle.

The freshener 1 according to the present disclosure further comprises a cap 6. Said cap 6 can be coupled and uncoupled to the first mouth 4 of the container 2. More details regarding the cap 6 will be provided later in this description.

Preferably, the container 2 and the cap 6 are made of a plastic material.

The freshener 1 further comprises an absorbing element 10. The absorbing element 10 is connected in a single piece with the cap 6 and is inserted into the cavity 5 of the container 2 to impregnate the liquid freshener composition. For example, the absorbing element 10 is a wick or cloth.

It should be noted that said absorbing element 10 is at least partially extractable from the cavity 5 through the first mouth 4 to allow the liquid freshener composition to evaporate from the absorbing element 10.

In greater detail, the cap 6 comprises a body 7. Said body 7 has a lower portion 61, wherein it is coupled to the first mouth 4, and an upper portion 62. Preferably, the body 7 comprises an internally threaded ferrule. In such a case, the first mouth 4 has an externally threaded surface that can be coupled to the body 7. The body 7 of the cap 6 can be coupled to the first mouth 4 by other closure methods known per se.

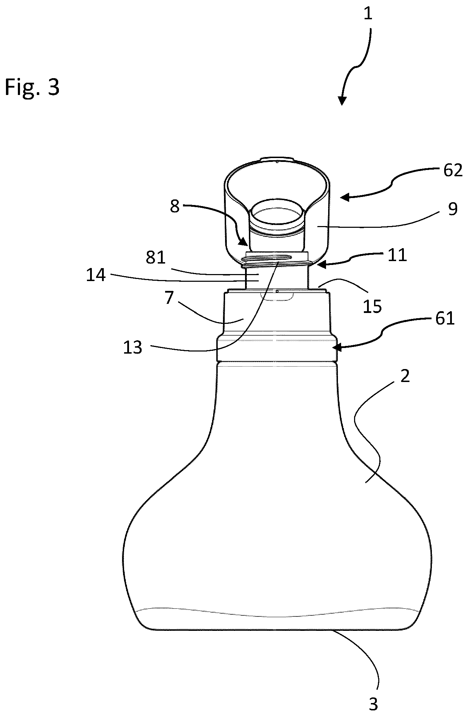

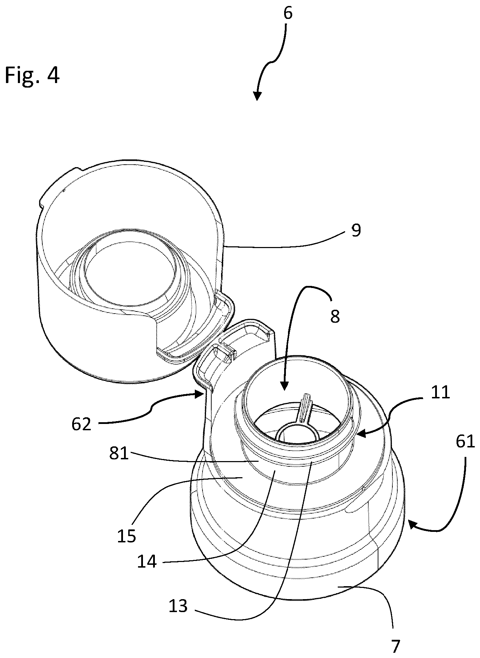

With particular reference to Figures 3 and 4, the body 7 of the cap 6 comprises a second mouth 8. Said second mouth 8 is placed in fluid communication with the cavity 5 of the container 2 when the cap 6 is coupled to the container 2.

The second mouth 8 has an outer surface 14 and an inner surface.

The cap 6 further comprises a cover 9, placed at the upper portion 62 for closing the second mouth 8. Said cover 9 is removable to allow access to the second mouth 8.

According to the invention the cover 9 is hinged to the body 7 in a peripheral position. For example, the cover 9 makes with the body 7 of the cap 6 a flip-top type closure. Alternatively, not according to the invention, the cover 9 is coupled to the body 7 by means of a snap-on mechanism or by screwing onto the body 7 which is in turn suitably shaped.

In particular, the second mouth 8 comprises connecting means 11 configured to reversibly couple the second mouth 8 with a capsule 12 configured to carry out a refill of the liquid freshener composition.

The capsule 12 is shaped to engage with the second mouth 8 by means of the connecting means 11 of the second mouth 8. Preferably, the connecting means 11 comprise a seat adapted to accommodate the capsule 12. The capsule 12 may, for example, be interlocked on the second mouth 8. According to a preferred embodiment, the connecting means 11 are embodied in a thread 13. Preferably, the thread 13 is placed at the outer surface 14 of the second mouth 8. Alternatively, the thread 13 is placed on the inner surface of the second mouth 8. In accordance, the capsule 12 is in turn internally or externally threaded to be connected to the thread 13 present on the second mouth 8 of the cap 6.

It should be noted that the connecting means 11 may embody connecting means alternative to the thread 13, the latter being the preferred embodiment of the present invention. Clearly, the connecting means 11 are complementary to the respective connecting means of the refill capsule 12. For example, the connecting means 11 may comprise bayonet or mechanical interlocking engagement/disengagement means.

Preferably, the body 7 comprises a base 15 placed at the upper portion 62. The second mouth 8 extends from the base 15 in axial direction moving away from the base 15 defining a neck 81. Always preferably, the thread 13 is placed at said neck 81, on the outer surface 14 or on the inner surface.

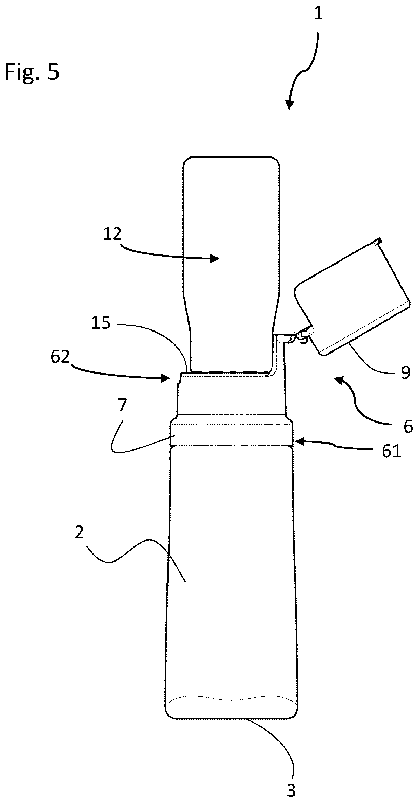

With reference to Figure 5, preferably, the capsule 12 abuts with the base 15 when coupled to the second mouth 8.

According to an embodiment, not illustrated, the body 7 comprises a notch at the upper portion 62. In accordance, the base 15 is obtained in the notch. According to this embodiment, the capsule 12 is inserted into the notch when coupled to the second mouth 8. Advantageously, accidental spillage of freshener composition during the refilling step is avoided.

The present invention relates to a kit. The kit comprises the freshener 1 described above and additionally at least one capsule 12. Said capsule 12 is configured to carry out a refill of the liquid freshener composition.

In particular, the capsule 12, also preferably made of a plastic material, contains within it a concentrated liquid or solid freshener composition. Said capsule 12 is shaped to release the freshener composition within the cavity 5 when coupled to the second mouth 8. For example, the capsule 12 comprises a frangible covering configured to face the second mouth 8. Said frangible covering is further configured to break when the capsule 12 is coupled to the second mouth 8 to release the content within the cavity.

Advantageously, in order to carry out a refilling step of the freshener 1, it is sufficient to lift the cover 9 from the body 7 and couple said capsule 12 to the second mouth 8. Advantageously, it is not necessary to uncouple the cap 6 from the first mouth 4 by also removing the absorbing element 10.

The present invention also relates to a method for refilling an air freshener 1 with a capsule 12 for carrying out a refill of the liquid freshener composition. Said method comprises a first step of providing the aforementioned kit. The kit comprises a freshener 1 and a capsule 12 for carrying out a refill of the liquid freshener composition.

The method thus comprises the step of removing the cover 9 from the body 7 of the cap 6. So, it is provided that the user couples the capsule 12 to the second mouth 8 of the cap 6 to pour the refill of freshener composition into the cavity 5.

The method thus comprises the step of uncoupling the capsule 12 from the second mouth 8. Thus, water can be supplied to the cavity 5 of the container 2 through the second mouth 8.

Finally, the method includes closing the cover 9 on the body 7 of the cap 6 and shaking the container 2 to mix the freshener composition refill with water.

In this way, the freshener 1 is made usable again.

## Claims

1. A kit comprising:
- an air freshener (1),
- at least a capsule (12) to carry out a refill of a liquid freshener composition containing a refill of freshener composition,
wherein the air freshener (1) comprises:
- a container (2) comprising a bottom (3) and one first mouth (4), said container (2) extending in an axial direction between the bottom (3) and the first mouth (4) defining a cavity (5) adapted to house the liquid freshener composition;
- a cap (6) that can be coupled and uncoupled to the first mouth (4) of the container (2);
- an absorbing element (10) connected in a single piece with the cap (6) and inserted into the cavity (5) of the container (2) to impregnate the liquid freshener composition, wherein by disengaging the cap (6) from the first mouth (4) the absorbing element (10) is at least partially extractable from the cavity (5) through the first mouth (4) to allow the liquid freshener composition to evaporate from the absorbing element (10);
wherein said cap (6) comprises:
- a body (7) comprising one second mouth (8) in fluid communication with the cavity (5) of the container (2) when the cap (6) is coupled to the container (2), said body (7) having a lower portion (61) wherein it is coupled to the first mouth (4) and an upper portion (62), said body (7) comprising a base (15) placed at the upper portion (62), said second mouth (8) extending from the base (15) in axial direction moving away from the base (15);
- a cover (9) placed at the upper portion (62) for closing the second mouth (8) and liftable to allow access to said second mouth (8);
- said second mouth (8) comprises connecting means (11) configured to reversibly couple the second mouth (8) with the capsule (12) configured to carry out a refill of the liquid freshener composition;
**characterized in that** the cover (9) is hinged to the body (7) in a peripheral position.

2. The kit according to claim 1, wherein said connecting means (11) comprise a thread (13).

3. The kit according to claim 2, wherein the second mouth (8) has an outer surface (14), the thread (13) being placed at the outer surface (14).

4. The kit according to any one of the preceding claims, wherein said body (7) comprises a notch at the upper portion (62), said base (15) being obtained in said notch.

5. The kit according to any one of the preceding claims, wherein the absorbing element (10) is a wick or a cloth.

6. The kit according to any one of the preceding claims, wherein the container (2) is a bottle.

7. A method for refilling an air freshener (1) with a capsule (12) to carry out a refill of the liquid freshener composition, said method comprising the steps of
- providing a kit according to any of the preceding claims;
- lifting the cover (9) from the body (7) of the cap (6);
- coupling the capsule (12) to the second mouth (8) of the cap (6) to pour the refill of freshener composition into the cavity (5);
- uncoupling the capsule (12) from the second mouth (8);
- providing water in the cavity (5) of the container (2) through the second mouth (8);
- closing the cover (9) on the body (7) of the cap (6);
- shaking the container (2) to mix the refill of freshener composition with water.

## Patentansprüche

1. Ein Kit, umfassend:
- einen Lufterfrischer (1),
- mindestens eine Kapsel (12) zum Durchführen eines Nachfüllens einer flüssigen Erfrischerzusammensetzung, die eine Nachfüllung von Erfrischerzusammensetzung enthält, wobei der Lufterfrischer (1) umfasst:
- einen Behälter (2), der einen Boden (3) und eine erste Mündung (4) umfasst, wobei sich der Behälter (2) in einer axialen Richtung zwischen dem Boden (3) und der ersten Mündung (4) erstreckt und einen Hohlraum (5) definiert, der dazu eingerichtet ist, die flüssige Erfrischerzusammensetzung aufzunehmen;
- eine Kappe (6), die mit der ersten Mündung (4) des Behälters (2) gekoppelt und von dieser entkoppelt werden kann;
- ein absorbierendes Element (10), das einstückig mit der Kappe (6) verbunden und in den Hohlraum (5) des Behälters (2) eingesetzt ist, um die flüssige Erfrischerzusammensetzung zu imprägnieren, wobei durch Lösen der Kappe (6) von der ersten Mündung (4) das absorbierende Element (10) zumindest teilweise durch die erste Mündung (4) aus dem Hohlraum (5) herausziehbar ist, um zu ermöglichen, dass die flüssige Erfrischerzusammensetzung von dem absorbierenden Element (10) verdampft; wobei die Kappe (6) umfasst:
- einen Körper (7), der eine zweite Mündung (8) umfasst, die in Fluidverbindung mit dem Hohlraum (5) des Behälters (2) steht, wenn die Kappe (6) mit dem Behälter (2) gekoppelt ist, wobei der Körper (7) einen unteren Abschnitt (61), der mit der ersten Mündung (4) gekoppelt ist, und einen oberen Abschnitt (62) aufweist, wobei der Körper (7) eine an dem oberen Abschnitt (62) angeordnete Basis (15) umfasst, wobei sich die zweite Mündung (8) in axialer Richtung von der Basis (15) weg erstreckt;
- eine Abdeckung (9), die an dem oberen Abschnitt (62) zum Verschließen der zweiten Mündung (8) angeordnet und anhebbar ist, um den Zugang zu der zweiten Mündung (8) zu ermöglichen;
- wobei die zweite Mündung (8) Verbindungsmittel (11) umfasst, die konfiguriert sind, um die zweite Mündung (8) reversibel mit der Kapsel (12) zu koppeln, die konfiguriert ist, um ein Nachfüllen der flüssigen Erfrischerzusammensetzung durchzuführen; **dadurch gekennzeichnet, dass** die Abdeckung (9) an einer peripheren Position gelenkig mit dem Körper (7) verbunden ist.

2. Kit nach Anspruch 1, wobei die Verbindungsmittel (11) ein Gewinde (13) umfassen.

3. Kit nach Anspruch 2, wobei die zweite Mündung (8) eine Außenfläche (14) aufweist, wobei das Gewinde (13) an der Außenfläche (14) angeordnet ist.

4. Kit nach einem der vorhergehenden Ansprüche, wobei der Körper (7) eine Einkerbung an dem oberen Abschnitt (62) umfasst, wobei die Basis (15) in der Einkerbung ausgebildet ist.

5. Kit nach einem der vorhergehenden Ansprüche, wobei das absorbierende Element (10) ein Docht oder ein Tuch ist.

6. Kit nach einem der vorhergehenden Ansprüche, wobei der Behälter (2) eine Flasche ist.

7. Ein Verfahren zum Nachfüllen eines Lufterfrischers (1) mit einer Kapsel (12) zum Durchführen eines Nachfüllens der flüssigen Erfrischerzusammensetzung, wobei das Verfahren die folgenden Schritte umfasst:
- Bereitstellen eines Kits nach einem der vorhergehenden Ansprüche;
- Anheben der Abdeckung (9) von dem Körper (7) der Kappe (6);
- Koppeln der Kapsel (12) mit der zweiten Mündung (8) der Kappe (6), um die Nachfüll-Erfrischerzusammensetzung in den Hohlraum (5) zu gießen;
- Entkoppeln der Kapsel (12) von der zweiten Mündung (8);
- Einbringen von Wasser in den Hohlraum (5) des Behälters (2) durch die zweite Mündung (8);
- Schließen der Abdeckung (9) auf dem Körper (7) der Kappe (6);
- Schütteln des Behälters (2), um die Nachfüll-Erfrischerzusammensetzung mit Wasser zu mischen.

## Revendications

1. Kit comprenant :
- un diffuseur de parfum (1),
- au moins une capsule (12) pour effectuer une recharge d'une composition de diffuseur de parfum liquide contenant une recharge de composition de diffuseur de parfum,
dans lequel le diffuseur de parfum (1) comprend:
- un récipient (2) comprenant un fond (3) et une première embouchure (4), ledit récipient (2) s'étendant dans une direction axiale entre le fond (3) et la première embouchure (4) en définissant une cavité (5) adaptée pour loger la composition de diffuseur de parfum liquide ;
- un bouchon (6) qui peut être couplé à et découplé de la première embouchure (4) du récipient (2) ;
- un élément absorbant (10) relié d'un seul tenant au bouchon (6) et inséré dans la cavité (5) du récipient (2) pour imprégner la composition de diffuseur de parfum liquide, dans lequel, en désengageant le bouchon (6) de la première embouchure (4), l'élément absorbant (10) est au moins partiellement extractible de la cavité (5) à travers la première embouchure (4) pour permettre à la composition de diffuseur de parfum liquide de s'évaporer depuis l'élément absorbant (10) ; dans lequel ledit bouchon (6) comprend :
- un corps (7) comprenant une seconde embouchure (8) en communication fluidique avec la cavité (5) du récipient (2) lorsque le bouchon (6) est couplé au récipient (2), ledit corps (7) ayant une partie inférieure (61) couplée à la première embouchure (4) et une partie supérieure (62), ledit corps (7) comprenant une base (15) placée au niveau de la partie supérieure (62), ladite seconde embouchure (8) s'étendant depuis la base (15) dans une direction axiale en s'éloignant de la base (15) ;
- un couvercle (9) placé au niveau de la partie supérieure (62) pour fermer la seconde embouchure (8) et pouvant être soulevé pour permettre l'accès à ladite seconde embouchure (8) ;
- ladite seconde embouchure (8) comprend des moyens de connexion (11) configurés pour coupler de manière réversible la seconde embouchure (8) à la capsule (12) configurée pour effectuer une recharge de la composition de diffuseur de parfum liquide ; **caractérisé en ce que** le couvercle (9) est articulé sur le corps (7) dans une position périphérique.

2. Kit selon la revendication 1, dans lequel lesdits moyens de connexion (11) comprennent un filetage (13).

3. Kit selon la revendication 2, dans lequel la seconde embouchure (8) a une surface extérieure (14), le filetage (13) étant placé sur la surface extérieure (14).

4. Kit selon l'une quelconque des revendications précédentes, dans lequel ledit corps (7) comprend une encoche au niveau de la partie supérieure (62), ladite base (15) étant réalisée dans ladite encoche.

5. Kit selon l'une quelconque des revendications précédentes, dans lequel l'élément absorbant (10) est une mèche ou une toile.

6. Kit selon l'une quelconque des revendications précédentes, dans lequel le récipient (2) est un flacon.

7. Procédé pour recharger un diffuseur de parfum (1) avec une capsule (12) pour effectuer une recharge de la composition de diffuseur de parfum liquide, ledit procédé comprenant les étapes consistant à :
- fournir un kit selon l'une quelconque des revendications précédentes ;
- soulever le couvercle (9) du corps (7) du bouchon (6) ;
- coupler la capsule (12) à la seconde embouchure (8) du bouchon (6) pour verser la recharge de composition de diffuseur de parfum dans la cavité (5) ;
- découpler la capsule (12) de la seconde embouchure (8) ;
- introduire de l'eau dans la cavité (5) du récipient (2) à travers la seconde embouchure (8) ;
- refermer le couvercle (9) sur le corps (7) du bouchon (6) ;
- agiter le récipient (2) pour mélanger la recharge de composition de diffuseur de parfum avec de l'eau.
